# EUROPÄISCHE PATENTSCHRIFT

(11) **EP 3 134 026 B1**
(45) Veröffentlichungstag und Bekanntmachung des Hinweises auf die Patenterteilung: **27.06.2018**
(21) Anmeldenummer: 15712029.6
(22) Anmeldetag: 04.02.2015
(51) Int. Cl.: A61C 8/02, A61C 8/00, A61F 2/28

(54) **ZAHNIMPLANTATSYSTEM**
DENTAL IMPLANT SYSTEM
SYSTÈME D'IMPLANT DENTAIRE

(30) Priorität: 15.04.2014 AT 2802014
(43) Veröffentlichungstag der Anmeldung: 01.03.2017
(73) Patentinhaber: Sonnleitner, Dietmar, 5020 Salzburg (AT)
(72) Erfinder: Sonnleitner, Dietmar, 5020 Salzburg (AT)
(74) Vertreter: Torggler & Hofinger Patentanwälte
(86) Internationale Anmeldenummer: PCT/AT2015/000016
(87) Internationale Veröffentlichungsnummer: WO 2015/157784

(56) Entgegenhaltungen:
- EP-A1- 0 890 345
- WO-A2-2008/021921
- KR-B1- 101 053 052
- US-B1- 6 171 106

## Beschreibung

Die Erfindung betrifft ein Zahnimplantatsystem gemäß dem Oberbegriff des Anspruchs 1.

Zahnimplantatsysteme, die unter Einsatz von Membrantechnik die Knochenregeneration einer Knochendefektstelle begünstigen, sind bereits bekannt (z.B. EP 0 890 345 A1, US 6 171 106 B1, KR 101 053 052 B1). Solche Zahnimplantatsysteme umfassen ein Implantat, das im Bereich der Knochendefektstelle im Kieferknochen verankert wird, eine die Knochenregeneration ermöglichende Folie bzw. Membran, die über die Knochendefektstelle und somit auch über das Implantat gespannt und am Kieferknochen befestigt wird, sowie eine Befestigungsschraube, deren Schraubenbolzen bei der Anbringung und lagestabilen Befestigung der Folie relativ zum Implantat durch die Folie hindurch ragt und mit einer Gewindebohrung des Implantats verschraubt wird. Zwischen der Folie und einer Oberfläche der Knochendefektstelle wird dadurch ein Hohlraum geschaffen, in dem Knochenmaterial und bei natürlichen Zähnen auch der Zahnhalteapparat nachwachsen kann. Für eine günstige Knochenregeneration kann der Hohlraum auch Knochenersatzmaterialien, Träger für Medikamente, Wachstumsfaktoren oder sonstige die Heilung und Knochenbildung fördernde und schützende Substanzen enthalten. Je nach Ausbildung der Knochendefektstelle kann es bei den bekannten Zahnimplantatsystemen beim Anlegen der Folie bzw. Membran zu einer unerwünschten Falten- oder Kraterbildung kommen, die wiederum zu einer unerwünschten Oberflächenstruktur des regenerierten Kieferknochens führen kann.

Aufgabe der Erfindung ist es daher, ein verbessertes Zahnimplantatsystem für die Knochenregeneration einer Knochendefektstelle eines Kieferknochens anzugeben, das insbesondere das Anlegen der Folie an der Knochendefektstelle erleichtert und eine verbesserte Oberflächenstruktur des regenerierten Knochenmaterials ermöglicht.

Diese Aufgabe wird erfindungsgemäß durch die Merkmale des Anspruchs 1 gelöst. Vorteilhafte Ausgestaltungen der Erfindung sind in den abhängigen Ansprüchen angegeben.

Gemäß der Erfindung ist also vorgesehen, dass ein Distanzelement zwischen dem Implantat und der Folie anordenbar ist, wobei in Montageposition der Schraubenbolzen durch das Distanzelement hindurch ragt. Durch das Vorsehen eines Distanzelements zwischen einem stirnseitigen Implantatkopf des Implantats und der über die Knochendefektstelle zu spannenden Folie kann ein unerwünschter Niveauunterschied zwischen einem ersten Niveau des Implantatkopfes und einem zweiten Niveau eines Randes der Knochendefektstelle abhängig von der Ausgestaltung der Knochendefektstelle in einfacher Weise ausgeglichen werden. Der Schraubenbolzen ragt dabei durch das Distanzelement hindurch und kann mit einem Innengewinde der Gewindebohrung des Implantats verschraubt werden. Dadurch, dass der Schraubenbolzen ohne Eingriff mit dem Distanzelement durch dieses hindurch ragt, dreht sich das Distanzelement während der lagestabilen Befestigung der Folie nicht mit der Befestigungsschraube mit, wodurch auch die Folie nicht verdreht oder verknittert wird. Somit lassen sich mit dem vorgeschlagenen Zahnimplantatsystem zwei wesentliche Vorteile erzielen. Zum einen kann durch die flexible Anpassung an gegebene Niveauunterschiede zwischen Implantatkopf und Rand der Knochendefektstelle eine unerwünschte Kraterbildung der Folie vermieden werden und zum anderen kann eine unerwünschte Faltenbildung im Bereich der Folie, in dem der Schraubenbolzen durch die Folie hindurch ragt, vermieden werden, da die Folie bei ihrer lagestabilen Befestigung nicht verdreht oder verknittert wird.

Gemäß einer bevorzugten Ausführungsform kann vorgesehen sein, dass das Distanzelement zum Hindurchführen des Schraubenbolzens eine Durchgangsbohrung mit einem Bohrungsdurchmesser aufweist, der größer ist als ein Außendurchmesser des Schraubenbolzens. Dadurch ragt der Schraubenbolzen berührungsfrei durch das Distanzelement hindurch und steht während der Verschraubung mit der Gewindebohrung des Implantats nicht im Eingriff mit dem Distanzelement. Dies stellt bereits eine einfache Verdrehsicherung für das Distanzelement dar.

Eine besonders bevorzugte Ausführungsvariante sieht vor, dass das Distanzelement am Implantat rotationsgesperrt anordenbar ist. Vorzugsweise kann dabei vorgesehen sein, dass als Rotationssperre am Distanzelement Vorsprünge angeordnet sind, die in korrespondierende Ausnehmungen des Implantats einführbar sind. Eine rotationsgesperrte Anordnung des Distanzelements am Implantat stellt sicher, dass sich das Distanzelement während der Befestigung der Folie relativ zum Implantat, das heißt während der Verschraubung der Befestigungsschraube mit dem Implantat, nicht mit der Befestigungsschraube mitdreht. Es kann somit eine Verdrehsicherung bereitgestellt werden, die eine unerwünschte Faltenbildung bei der Befestigung der Folie verhindert.

Besonders vorteilhaft ist jene Ausführungsform der Erfindung, bei der das Distanzelement im Wesentlichen hohlzylinderförmig, vorzugsweise als im Wesentlichen rohrförmige Hülse, ausgebildet ist. Dabei kann das Distanzelement als besonders einfach zu fertigende Distanzhülse ausgebildet sein.

Es kann auch vorgesehen sein, dass das Distanzelement im Wesentlichen konisch oder kegelstumpfförmig ausgebildet ist. Dadurch kann insbesondere eine vergrößerte Auflagefläche für die Folie am Distanzelement gebildet werden und die anatomische Form einer natürlichen Wurzel nachgebildet werden.

Gemäß einer bevorzugten Ausführung kann vorgesehen sein, dass die Befestigungsschraube einen Schraubenkopf aufweist, wobei die Folie zwischen Distanzelement und Schraubenkopf einklemmbar ist. Dabei kann der Schraubenkopf der Befestigungsschraube einen sogenannten Heilpfosten bilden, der als Platzhalter für einen nach der Knochenregenration anzubringenden Durchtrittspfosten dient, der einen Kanal durch das Zahnfleisch in die Mundhöhle offen hält.

Um eine Faltenbildung der Folie weiter zu verringern, kann vorzugsweise vorgesehen sein, dass der Schraubenkopf an einer in Montageposition der Folie zugewandten Schraubenkopfoberfläche mit einer reibungsverringernden Beschichtung, vorzugsweise mit einer Teflonbeschichtung, versehen ist. Es kann natürlich auch vorgesehen sein, dass die Folie an einer in Montageposition dem Schraubenkopf zugewandten Folienoberfläche wenigstens in einem Kontaktbereich mit dem Schraubenkopf mit einer reibungsverringernden Beschichtung, vorzugsweise mit einer Teflonbeschichtung, versehen ist.

Um auch eine flexible Ausgestaltung der Höhe des einzusetzenden Heilpfostens zu ermöglichen, ist vorgesehen, dass das Zahnimplantatsystem eine im Wesentlichen rohrförmige Befestigungshülse umfasst, durch die in Montagposition der Schraubenbolzen hindurch ragt, wobei die Folie zwischen Distanzelement und Befestigungshülse einklemmbar ist. Die Befestigungshülse kann hierbei den Heilpfosten bilden, wodurch in einfacher Weise verschiedene Höhen von Heilpfosten bereitgestellt werden können.

Vorzugsweise kann dabei vorgesehen sein, dass die Befestigungshülse zum Hindurchführen des Schraubenbolzens ein Durchgangsloch mit einem Lochdurchmesser aufweist, der größer ist als ein Außendurchmesser des Schraubenbolzens. Dabei ragt der Schraubenbolzen berührungsfrei durch die Befestigungshülse hindurch, wodurch sich die Befestigungshülse während der Befestigung der Befestigungsschraube am Implantat nicht mit der Befestigungsschraube mitdreht und es somit zu keiner unerwünschten Verdrehung oder Faltenbildung der Folie kommt.

Eine besondere Ausführungsvariante sieht dabei vor, dass an der Befestigungshülse an einer in Montageposition der Folie zugewandten Hülsenoberfläche wenigstens ein - vorzugsweise im Wesentlichen dornartig ausgebildeter - Fortsatz zur Lagefixierung der Folie relativ zur Befestigungshülse angeordnet ist. Dadurch kann ein Verdrehen oder Knittern der Folie verhindert werden.

Gemäß einer besonders bevorzugten Ausführung kann vorgesehen sein, dass die Folie im Wesentlichen vollständig resorbierbar ist. Dadurch, dass die Folie oder Membran insgesamt vollständig im Körper resorbierbar ist, indem sie beispielweise durch Hydrolyse im Körper abgebaut wird, entfällt das Durchführen einer weiteren Operation zur Entfernung der Folie nach erfolgter Knochenregeneration.

Besonders vorteilhaft ist jene Ausführungsform, bei der die Folie eine, vorzugsweise vorverbundene, mehrschichtige Folie ist, die eine formgebende Formschicht zur
Anformung der Folie an die Knochendefektstelle und wenigstens eine Deckschicht zur Abdeckung der Knochendefektstelle umfasst, wobei die Formschicht und die wenigstens eine Deckschicht im Wesentlichen vollständig resorbierbar sind. Hierbei umfasst die Folie eine formgebende Formschicht, die der Anformung der Folie an die Knochendefektstelle dient und durch die ein Hohlraum zwischen Knochendefektstelle und Folie gebildet werden kann, sodass in diesem Hohlraum Knochenwachstum stattfinden kann. Der Hohlraum wird durch die raumbildende und raumhaltende Formschicht solange aufrechterhalten, bis der Hohlraum durch nachwachsendes Knochenmaterial ausgefüllt ist. Darüber hinaus umfasst die Folie dieser Ausführungsform wenigstens eine Deckschicht zur Abdeckung der Knochendefektstelle. Diese Deckschicht, die beispielsweise als Membran ausgebildet sein kann, dient zur Überdeckung und Abdichtung der Knochendefektstelle, um das Eindringen von Weichgewebe in die Knochendefektstelle zu vermeiden. Um die Anbringung der Folie und die Abdichtung der Knochendefektstelle weiter zu verbessern, kann die wenigstens eine Deckschicht auch so ausgebildet sein, dass sie mit einem die Knochendefektstelle umgebenden Zahnfleisch verklebt. Die einzelnen Schichten einer solchen vorverbundenen Folie (Formschicht und wenigstens eine Deckschicht) können mechanisch und/oder chemisch miteinander verbunden sein.

Gemäß einer bevorzugten Ausführungsform kann vorgesehen sein, dass die Formschicht und die wenigstens eine Deckschicht in unterschiedlichen Zeiträumen im Wesentlichen vollständig resorbierbar sind. So kann beispielsweise durch Auslegung der Formschicht und der wenigstens einen Deckschicht erreicht werden, dass die Formschicht schneller resorbiert als die wenigstens eine Deckschicht. Generell ergeben sich durch unterschiedlich starke Resorbierbarkeiten der Formschicht und der wenigstens einen Deckschicht große Freiheiten in der Auslegung der Folie in Bezug auf ihre Resorbierbarkeit.

Es kann vorgesehen sein, dass die Folie insgesamt in einem Zeitraum von etwa 3 bis 12 Monaten, vorzugsweise etwa 4 bis 6 Monaten, im Wesentlichen vollständig resorbierbar ist. Dies ist der Zeitraum innerhalb dessen im Normalfall der Knochenwiederaufbau erfolgt ist.

Um eine gute Anformung an die Knochendefektstelle und eine stabile Hohlraumbildung zwischen Folie und Knochendefektstelle zu ermöglichen, kann vorgesehen sein, dass die Formschicht steifer als die wenigstens eine Deckschicht ausgebildet ist. Die höhere Steifigkeit der Formschicht dient dabei dazu, einen Hohlraum für den Knochenaufbau zu bilden und diesen Hohlraum auch für den für die Knochenregeneration benötigten Zeitraum aufrecht zu erhalten. Durch eine im Vergleich zur Formschicht geringeren Steifigkeit der wenigstens einen Deckschicht kann wiederum eine gute Überdeckung und Abdichtung der Knochendefektstelle erzielt werden.

Vorzugsweise kann vorgesehen sein, dass die Formschicht, gegebenenfalls zusammen mit der wenigstens einen Deckschicht, mechanisch und/oder thermisch und/oder chemisch verformbar ausgebildet ist. So kann insbesondere die Formschicht als im Wesentlichen formstabile Schicht ausgebildet sein, die sich unter mechanischem, thermischem oder chemischem Einfluss verformen lässt und nach dieser Verformung wiederum eine ausreichende Formstabilität aufweist, um den für das Knochenwachstum zu bildenden Hohlraum für die erforderliche Zeitspanne aufrecht zu erhalten. Die wenigstens eine Deckschicht kann flexibel und vorzugsweise elastisch sein, um eine gute Abdeckung und Abdichtung der Knochendefektstelle zu ermöglichen.

Eine mechanische Verformung kann dabei beispielsweise durch Verbiegen mit einer Zange erfolgen. Dies ist insbesondere für verhältnismäßig dünne Formschichten (z.B. im Bereich von etwa 0,10 mm bis etwa 0,5 mm) eine geeignete Methode der Anformung. Für dickere Formschichten (z.B. dicker als etwa 0,5 mm) kann eine thermische Verformung einer Formschicht zur Anformung zweckmäßig sein. Eine entsprechende thermische Verformung kann dabei beispielsweise mittels eines Thermostabes mit einer heißen Spitze oder Fläche, über erwärmte vorgefertigte Modelle oder im heißen Wasserbad mit steriler Kochsalzlösung erzielt werden.

Für eine gute Resorbierbarkeit der vorgeschlagenen Folie kann vorgesehen sein, dass die wenigstens eine Deckschicht zumindest teilweise, vorzugsweise im Wesentlichen vollständig, aus einem bioresorbierbaren Collagenmaterial besteht. Dabei kann vorgesehen sein, dass das bioresorbierbare Collagenmaterial Typ-I-Collagen und/oder Typ-III-Collagen umfasst. Das Collagenmaterial kann beispielsweise aus bovinen Achillessehnen stammen.

Für eine gute Resorbierbarkeit der vorgeschlagenen Folie kann darüber hinaus vorgesehen sein, dass die Formschicht zumindest teilweise, vorzugsweise im Wesentlichen vollständig, aus einem bioresorbierbaren Polymermaterial besteht. Bei dem bioresorbierbaren Polymermaterial kann es sich auch um ein Co-Polymermaterial handeln.

Eine besondere Ausführungsvariante sieht vor, dass das bioresorbierbare Polymermaterial Milchsäure, vorzugsweise L-Milchsäure, und/oder deren Derivate umfasst. Vorteilhaft ist es dabei, wenn der Anteil der Milchsäure im bioresorbierbaren Polymermaterial mindestens 70%, vorzugsweise etwa 80% bis 95%, besonders bevorzugt im Wesentlichen etwa 82%, beträgt.

Darüber hinaus kann vorgesehen sein, dass das bioresorbierbare Polymermaterial Glykolsäure umfasst. Vorteilhaft ist es dabei, wenn der Anteil der Glykolsäure im bioresorbierbaren Polymermaterial höchstens 30%, vorzugsweise etwa 15% bis 20%, besonders bevorzugt im Wesentlichen etwa 18%, beträgt. Je nach Zusammensetzung der Formschicht kann erreicht werden, dass die Formschicht im Wesentlichen formstabil und dennoch im Wesentlichen vollständig resorbierbar ist.

Bei einer weiteren bevorzugten Ausführungsform kann vorgesehen sein, dass die Formschicht und die wenigstens eine Deckschicht unterschiedliche Flächenausdehnungen aufweisen. Dabei kann vorgesehen sein, dass die Formschicht eine geringere Flächenausdehnung als die wenigstens eine Deckschicht einnimmt. Wenn die wenigstens eine Deckschicht die Formschicht aufgrund ihrer geringeren Flächenausdehnung überdeckt, kann eine besonders gute Abdeckung und somit auch Abdichtung der Knochendefektstelle erreicht werden.

Vorzugsweise kann vorgesehen sein, dass die wenigstens eine Deckschicht und/oder die Formschicht im Wesentlichen durchgehend flächig ausgebildet ist bzw. sind. Eine für die Anformung an die Knochendefektstelle günstige Kontur der Folie kann dabei beispielsweise durch entsprechendes Schneiden der Folie erreicht werden.

Besonders günstig ist es jedoch, wenn die Formschicht zur Anformung an die Knochendefektstelle eine Formstruktur aufweist. Dabei kann vorgesehen sein, dass die Formstruktur wenigstens abschnittsweise einen konvex und/oder konkav gekrümmten Rand und/oder wenigstens abschnittsweise eine konvex und/oder konkav gekrümmte Form aufweist. Mit anderen Worten kann die Formstruktur beispielsweise flächige - konvex und/oder konkav gekrümmte - Vorsprünge aufweisen und somit einen konvex und/oder konkav gekrümmten Rand aufweisen. Alternativ oder zusätzlich kann auch die Formstruktur als Ganzes eine entsprechend konvex und/oder konkav gekrümmte Form aufweisen.

Besonders vorteilhaft ist es, wenn die Formstruktur wenigstens ein strebenförmiges Anformelement aufweist. Die strebenförmigen oder lappenförmigen Anformelemente können dabei bügelartig über die Knochendefektstelle geformt werden und eine beliebige Hohlraumform ermöglichen.

Besonders vorteilhaft ist jene Ausführungsform der Erfindung, bei der die Formstruktur im Wesentlichen gitterförmig ausgebildet ist. Die gitterförmige Formstruktur bildet in diesem Fall ein Verstärkungsgitter, das die Bildung einer Vielzahl von beliebigen Hohlraumformen ermöglicht.

Es kann auch vorgesehen sein, dass die Formstruktur durch wenigstens eine Verstärkung der Formschicht ausgebildet ist. Insbesondere, wenn die Formschicht in Form einer aushärtenden Flüssigkeit oder eines aushärtenden Gels auf die wenigstens eine Deckschicht aufgebracht wird, ist es günstig, wenn die Formstruktur lediglich durch das Aufbringen von mehr Flüssigkeit oder Gel im Bereich der Formstruktur erzielt werden kann. In diesem Fall kann beispielsweise die Formschicht unterschiedliche Dicken aufweisen.

Eine besondere Ausführungsvariante sieht vor, dass die Folie eine Trägerschicht für wenigstens eine darauf anzuordnende oder angeordnete Substanz aufweist. Bei den auf der Trägerschicht anzuordnenden oder angeordneten Substanzen kann es sich dabei um Medikamente, Wachstumsfaktoren und/oder sonstige die Heilung und Knochenbildung fördernde und schützende Substanzen handeln. Die Trägerschicht kann vorzugsweise an einer der Knochendefektstelle zuzuwendenden Seite der Folie angeordnet sein und zumindest teilweise, vorzugsweise im Wesentlichen vollständig, aus einem bioresorbierbaren Collagenmaterial bestehen.

Es kann auch vorgesehen sein, dass entsprechende Substanzen direkt auf der Formschicht und/oder der wenigstens einen Deckschicht angebracht sind. Es kann auch vorgesehen sein, dass die einer Knochendefektstelle zuzuwendende Seite bzw. Oberfläche der Folie selbst als Träger für die oben beschriebenen Substanzen dient, indem beispielsweise diese Seite bzw. Oberfläche der Folie eine entsprechende Rauigkeit aufweist.

Die Folie oder Membran kann je nach Anwendungsfall auch vorgeschnitten und/oder vorgeformt bereitgestellt werden. Dabei kann beispielsweise ein gewünschter Zuschnitt und/oder eine gewünschte 3D-Verformung der Folie nach einer datenverarbeitungsgestützten Planung erfolgen.

Generell können die einzelnen Bestandteile des vorgeschlagenen Zahnimplantatsystems je nach Anwendungsfall individuell datenverarbeitungsgestützt geplant und gefertigt, beispielsweise gefräst, werden.

Weitere Einzelheiten und Vorteile der vorliegenden Erfindung werden anhand der nachfolgenden Figurenbeschreibung erläutert. Dabei zeigen
- Fig. 1a: eine Seitenansicht eines Implantats,
- Fig. 1b: eine Draufsicht auf das Implantat der Figur 1a,
- Fig. 2a: eine Seitenansicht des vorgeschlagenen Distanzelements, das als Distanzhülse ausgebildet ist,
- Fig. 2b: eine Draufsicht auf das Distanzelement der Figur 2a,
- Fig. 3a: eine Seitenansicht einer Folie zur Überdeckung einer Knochendefektstelle und eines Implantats,
- Fig. 3b: eine Draufsicht auf die Folie der Figur 3a,
- Fig. 4: eine Seitenansicht einer Befestigungsschraube,
- Fig. 5: ein Ausführungsbeispiel des vorgeschlagenen Zahnimplantatsystems während der Befestigung an einer Knochendefektstelle eines Kieferknochens,
- Fig. 6 - 8: mehrere Ausführungsbeispiele des vorgeschlagenen Zahnimplantatsystems, jeweils angeordnet an einer Knochendefektstelle eines Kieferknochens,
- Fig. 9: eine Schnittdarstellung durch eine regenerierte Knochendefektstelle,
- Fig. 10: ein Ausführungsbeispiel einer mehrschichtigen Folie in einer perspektivischen Explosionsdarstellung,
- Fig. 11: eine Seitenansicht der mehrschichtigen Folie gemäß Figur 10,
- Fig. 12 - 16: Draufsichten auf verschiedene Ausführungsformen von mehrschichtigen Folien und
- Fig. 17 - 24: mehrere Ausführungsbeispiele von mehrschichtigen Folien in perspektivischen Explosionsdarstellungen.

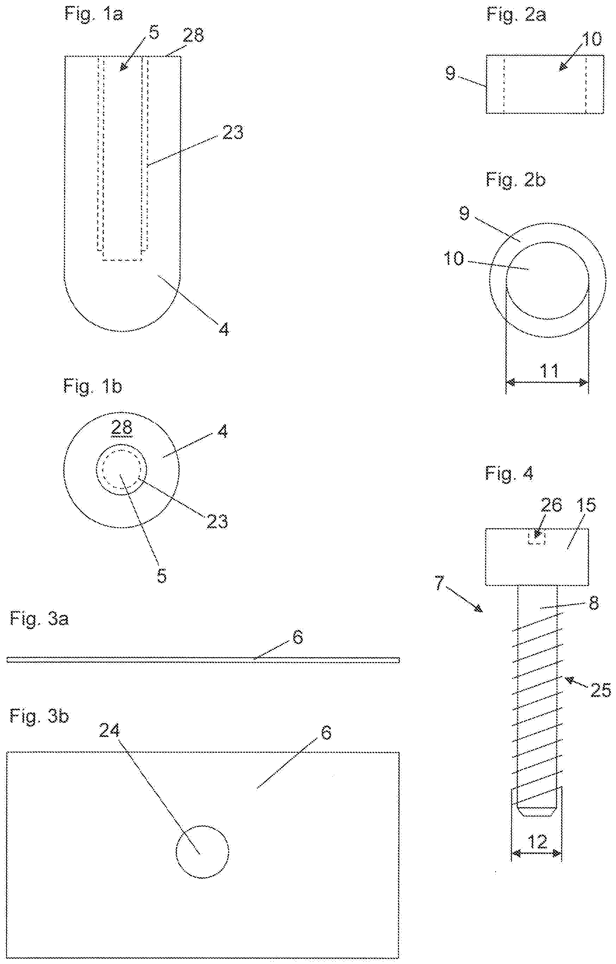

Figur 1a zeigt eine Seitenansicht eines Implantats 4 eines vorgeschlagenen Zahnimplantatsystems 1 und Figur 1b zeigt eine Draufsicht auf das Implantat 4. Das Implantat 4 weist ausgehend von Implantatkopf 28 des Implantats 4 eine Gewindebohrung 5 auf, die mit einem Innengewinde 23 versehen ist.

Figur 2a zeigt eine Seitenansicht auf ein Distanzelement 9 eines vorgeschlagenen Zahnimplantatsystems 1 und Figur 2b zeigt eine Draufsicht auf das Distanzelement 9. Das Distanzelement 9 ist in diesem Beispiel als Distanzhülse ausgebildet und weist eine Durchgangsbohrung 10 mit einem Bohrungsdurchmesser 11 auf.

Figur 3a zeigt eine Seitenansicht einer Folie 6 eines vorgeschlagenen Zahnimplantatsystems 1 und Figur 3b zeigt eine Draufsicht auf die Folie 6. Die Folie 6 weist ein Loch 24 auf, durch das in Montageposition ein Schraubenbolzen 8 einer Befestigungsschraube 7 hindurchgeführt werden kann.

Figur 4 zeigt eine Seitenansicht einer Befestigungsschraube 7 eines vorgeschlagenen Zahnimplantatsystems 1. Die Befestigungsschraube 7 umfasst einen Schraubenkopf 15 und einen daran angeordneten Schraubenbolzen 8, der mit einem Gewinde 25 ausgestattet ist. Schraubenbolzen 8 mit Gewinde 25 und Gewindebohrung 5 mit Innengewinde 23 sind so dimensioniert, dass das Gewinde 25 des Schraubenbolzens 8 mit dem Innengewinde 23 der Gewindebohrung 5 verschraubbar ist. Der Schraubenbolzen 8 weist einen Außendurchmesser 12 auf, der kleiner ist als der Bohrungsdurchmesser 11 der Durchgangsbohrung 10 des Distanzelements 9. Der Schraubenkopf 15 weist eine Werkzeugausnehmung 26 auf, in die ein entsprechendes Werkzeug eingreifen kann, um die Befestigungsschraube 7 mit dem Implantat 4 zu verschrauben, wie beispielsweise ein Schraubendreher oder ein Sechskant-Inbusschlüssel.

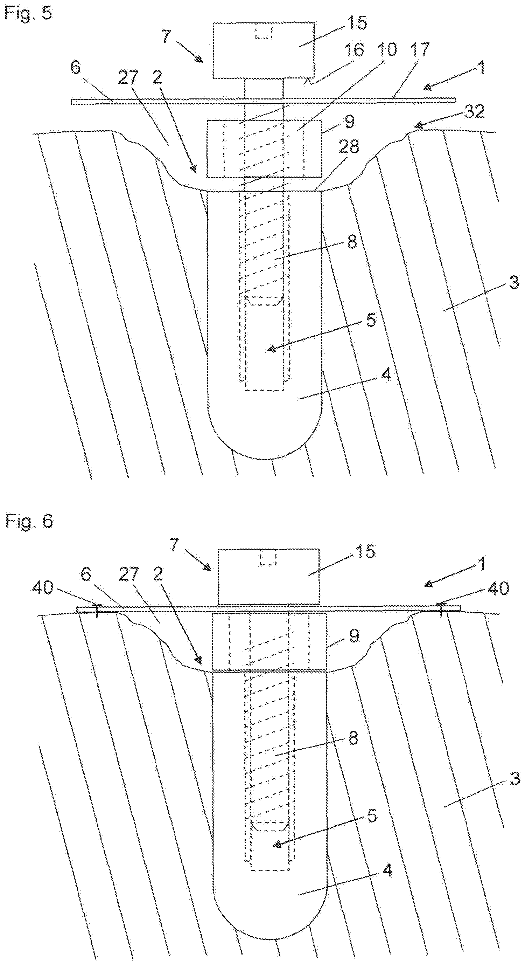

Figur 5 zeigt eine Schnittdarstellung durch eine Knochendefektstelle 2 eines Kieferknochens 3, wobei im Bereich der Knochendefektstelle 2 im Kieferknochen 3 ein Implantat 4 verankert ist. Um eine Knochenregeneration im Bereich der Knochendefektstelle 2 zu ermöglichen, wird über die Knochendefektstelle 2 und somit auch über das Implantat 4 eine Membran bzw. Folie 6 gespannt und am Kieferknochen 3 befestigt, um einen Hohlraum 27 zwischen Knochendefektstelle 2 und Folie 6 zu bilden, in dem sich der Kieferknochen 3 regenerieren kann. Je nach Ausbildung einer Knochendefektstelle 2 und Verankerung des Implantats 4 können sich jedoch unterschiedliche Niveauunterschiede zwischen einem ersten Niveau des Implantatkopfs 28 des Implantats 4 und einem zweiten Niveau eines Randes 32 der Knochendefektstelle 2 ergeben. Um trotz eines solchen Niveauunterschieds ein gleichmäßiges Knochenwachstum und eine erwünschte Oberfläche des regenerierten Kieferknochens 3 zu erhalten, ist ein Distanzelement 9 vorgesehen, das zwischen Implantat 4 und Folie 6 angeordnet wird, um eben diesen Niveauunterschied auszugleichen. Zur lagestabilen Befestigung der Folie 6 wird nun der Schraubenbolzen 8 einer Befestigungsschraube 7 durch die Folie 6 bzw. ein entsprechendes Loch 24 der Folie 6 sowie durch die Durchgangsbohrung 10 des Distanzelements 9 hindurchgeführt und mit der Gewindebohrung 5 des Implantats 4 verschraubt. Die Durchgangsbohrung 10 des Distanzelements 9 weist einen Bohrungsdurchmesser 11 auf, der größer ist als ein Außendurchmesser 12 des Schraubenbolzens 8 (siehe auch Figur 2b und Figur 4). Dadurch ist der Schraubenbolzen 8 während des Verschraubens der Befestigungsschraube 7 nicht mit dem Distanzelement 9 in Eingriff und das Distanzelement 9 dreht sich nicht mit der Befestigungsschraube 7 mit. Im gezeigten Beispiel ist darüber hinaus eine der Folie 6 zugewandte Schraubenkopfoberfläche 16 des Schraubenkopfs 15 der Befestigungsschraube 7 mit einer reibungsverringernden Beschichtung, beispielsweise mit einer Teflonbeschichtung, versehen. Dadurch kann ein Mitdrehen der Folie 6 während ihrer lagestabilen Befestigung durch Einklemmen zwischen Schraubenkopf 15 und Distanzelement 9 vermieden werden, wodurch es in weiterer Folge zu keiner unerwünschten Faltenbildung der Folie 6 im Einklemmbereich kommt. Alternativ oder zusätzlich kann auch an einer Folienoberfläche 17 eine reibungsverringernde Beschichtung vorgesehen sein.

Figur 6 zeigt das Zahnimplantatsystem 1 der Figur 5 nach Verschraubung der Befestigungsschraube 7 mit dem Implantat 4. Durch das vorgesehene Distanzelement 9 kann eine gleichmäßige Abdeckung der Knochendefektstelle 2 mit der Folie 6 erzielt werden, ohne dass beispielsweise unerwünschte Falten oder Krater in der Folie 6 entstehen. Zusätzlich zum Einklemmen der Folie 6 zwischen Schraubenkopf 15 und Distanzelement 9 wird die Folie 6 mittels entsprechender Befestigungsvorrichtungen 40 am Kieferknochen 3 verankert. Bei den Befestigungsvorrichtungen 40 kann es sich beispielsweise um metallene oder resorbierbare Nägel, Pins oder Schrauben handeln, die durch die Folie 6 hindurch am Kieferknochen 3 befestigt werden. Alternativ kann die Folie 6 auch mit dem Kieferknochen 3 verklebt werden.

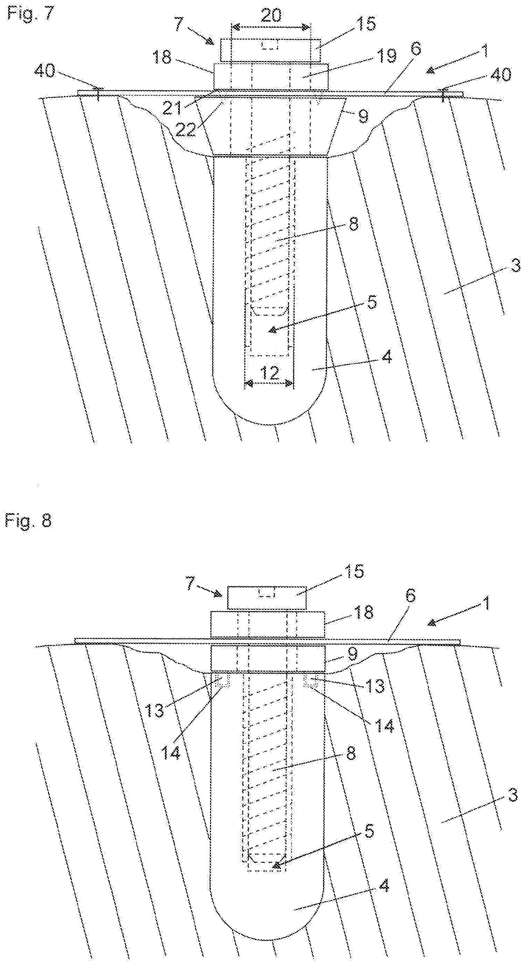

Figur 7 zeigt ein weiteres Beispiel eines vorgeschlagenen Zahnimplantatsystems 1 in einer Schnittdarstellung durch einen Kieferknochen 3. In diesem Beispiel weist das Distanzelement 9 eine konische bzw. kegelstumpfförmige Mantelfläche auf, durch die eine vergrößerte Auflagefläche für die Folie 6 bereitgestellt und die anatomische Wurzelform nachgebildet werden kann. Darüber hinaus ist eine Befestigungshülse 18 vorgesehen, die einen Kanal durch das Zahnfleisch zur Mundhöhle offenhält und die ein Durchgangsloch 19 mit einem Lochdurchmesser 20 aufweist, der größer ist als ein Außendurchmesser 12 des Schraubenbolzens 8 der Befestigungsschraube 7. Dadurch steht das Außengewinde des Schraubenbolzens 8 während des Verschraubens der Befestigungsschraube 7 mit der Befestigungshülse 18 nicht in Eingriff, wodurch sich die Befestigungshülse 18 nicht mit der Befestigungsschraube 7 mitdreht. Dadurch kann ein Mitdrehen der Folie 6 während ihrer lagestabilen Befestigung durch Einklemmen zwischen Befestigungshülse 18 und Distanzelement 9 vermieden werden, wodurch es in weiterer Folge zu keiner unerwünschten Faltenbildung der Folie 6 im Einklemmbereich kommt. An einer der Folie 6 zugewandten Hülsenoberfläche 21 der Befestigungshülse 18 sind darüber hinaus dornartige Fortsätze 22 angeordnet, welche die Folie 6 durchdringen und somit eine weitere Verdrehsicherung für die Folie 6 darstellen.

Figur 8 zeigt ein weiteres Ausführungsbeispiel eines vorgeschlagenen Zahnimplantatsystems 1 mit einem Distanzelement 9, einer Befestigungshülse 18 und einer zwischen Distanzelement 9 und Befestigungshülse 18 eingeklemmten Folie 6. In diesem Beispiel ist das Distanzelement 9 rotationsgesperrt am Implantat 4 angeordnet, indem vom Distanzelement 9 abstehende Vorsprünge 13 in korrespondierende Ausnehmungen 14 am Implantat 4 eingreifen. Die Folie 6 ist in diesem Beispiel an ihren Randbereichen am Kieferknochen 3 angeklebt.

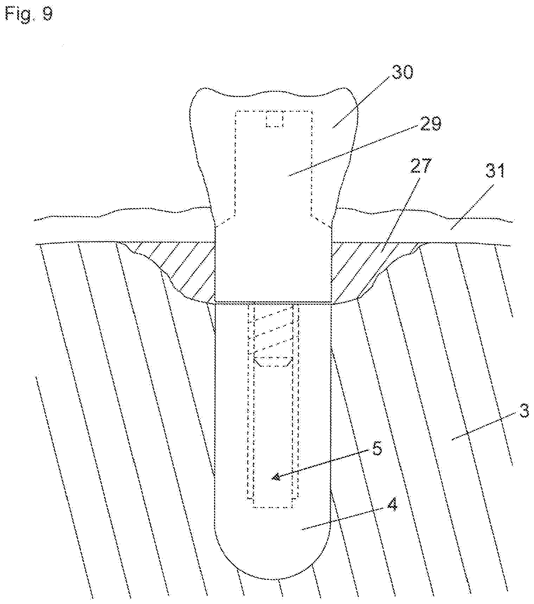

Figur 9 zeigt einen Kieferknochen 3 mit verankertem Implantat 4 nach erfolgter Knochenregeneration des Kieferknochens 3 im Hohlraum 27. Nach erfolgter Knochenregeneration werden die Befestigungsschraube 7, das Distanzelement 9 sowie gegebenenfalls die Folie 6 und die Befestigungshülse 18 entfernt und ein Durchtrittspfosten 29 am Implantat 4 angeordnet, der bis über das Zahnfleisch 31 in eine Mundhöhle ragt und an dem ein Zahnersatz 30 befestigt werden kann.

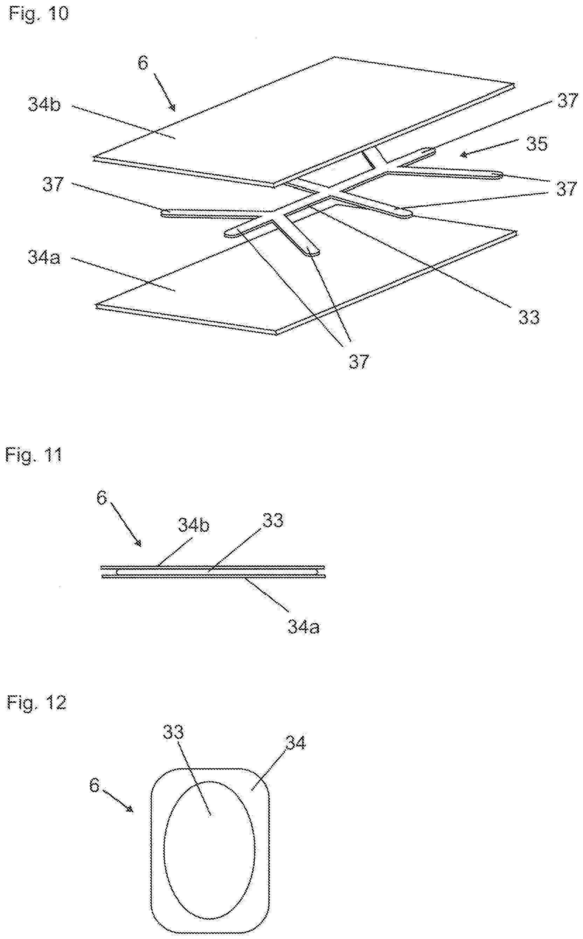

Figur 10 zeigt eine perspektivische Explosionsdarstellung einer vorgeschlagenen vorverbundenen mehrschichtigen Folie 6. Die Folie 6 umfasst eine Formschicht 33 sowie zwei Deckschichten 34a und 34b. Die Formschicht 33 ist steifer als die Deckschichten 34a und 34b ausgebildet und weist eine Formstruktur 35 auf. Die Formstruktur 35 umfasst mehrere strebenförmige Anformelemente 37, die dazu dienen, die Folie 6 über eine Knochendefektstelle 2 (hier nicht gezeigt) zu formen, wobei sich die Folie 6 durch die Anformelemente 37 gut an einen noch vorhandenen Kieferknochen 3 der Knochendefektstelle 2 anformen lässt. Die Formstruktur 35 ist insgesamt im Wesentlichen gitterförmig ausgebildet und ermöglicht somit die Ausbildung beliebiger Oberflächenformen der Folie 6, sodass sich in Verbindung mit einer Knochendefektstelle 2 beliebige Hohlraumformen zwischen Folie 6 und Knochendefektstelle 2 bilden lassen.

Die Formschicht 33 sowie die Deckschichten 34a und 34b bestehen jeweils aus einem bioresorbierbaren Material, sodass die Folie 6 insgesamt im Körper im Wesentlichen vollständig resorbierbar ist. Durch das Vorsehen von zwei Deckschichten 34a und 34b, zwischen denen die Formschicht 33 eingebettet ist, lässt sich insbesondere die Resorptionsgeschwindigkeit und mechanische Festigkeit der Formschicht 33 steuern.

Bei den Deckschichten 34a und 34b kann es sich beispielsweise um bioresorbierbare Collagen-Membranen handeln, die einerseits durch ihre Weichheit eine Knochendefektstelle 2 gut abdecken können und andererseits mit einem die Knochendefektstelle 2 umgebenden Zahnfleisch 31 gut verkleben können, sodass sich eine gute Abdichtung der Knochendefektstelle 2 ergibt.

Die Formschicht 33 kann beispielsweise aus einem bioresorbierbaren Polymermaterial oder Co-Polymermaterial bestehen. Insbesondere kann die Formschicht 33 beispielsweise etwa 82% L-Milchsäure und etwa 18% Glycolsäure umfassen. Eine solche Materialwahl ergibt eine im Wesentlichen formstabile Formschicht 33, die zur Anformung an eine Knochendefektstelle 2 mechanisch, thermisch und/oder chemisch verformbar ausgebildet sein kann, wobei die Formschicht 33 nach einer solchen Verformung im Wesentlichen wiederum formstabil ist. Aufgrund der Steifigkeit und Formstabilität der Formschicht 33 kann somit zwischen der Folie 6 und einer Knochendefektstelle 2 ein Hohlraum 27 für die Knochenregeneration geschaffen und für den Zeitraum der Knochenregeneration auch gehalten werden.

Figur 11 zeigt eine Seitenansicht der vorverbundenen mehrschichtigen Folie 6 gemäß Figur 10.

Figur 12 zeigt eine Draufsicht auf eine weitere Variante der vorgeschlagenen Folie 6, die in diesem Beispiel zweischichtig ausgeführt ist und eine Formschicht 33 und eine Deckschicht 34 umfasst. Sowohl die Formschicht 33 als auch die Deckschicht 34 sind im Wesentlichen flächig ausgebildet. Die Folie 6 kann beliebig zugeschnitten werden, um je nach Anwendungsfall eine gute Anformung an eine Knochendefektstelle 2 zu ermöglichen.

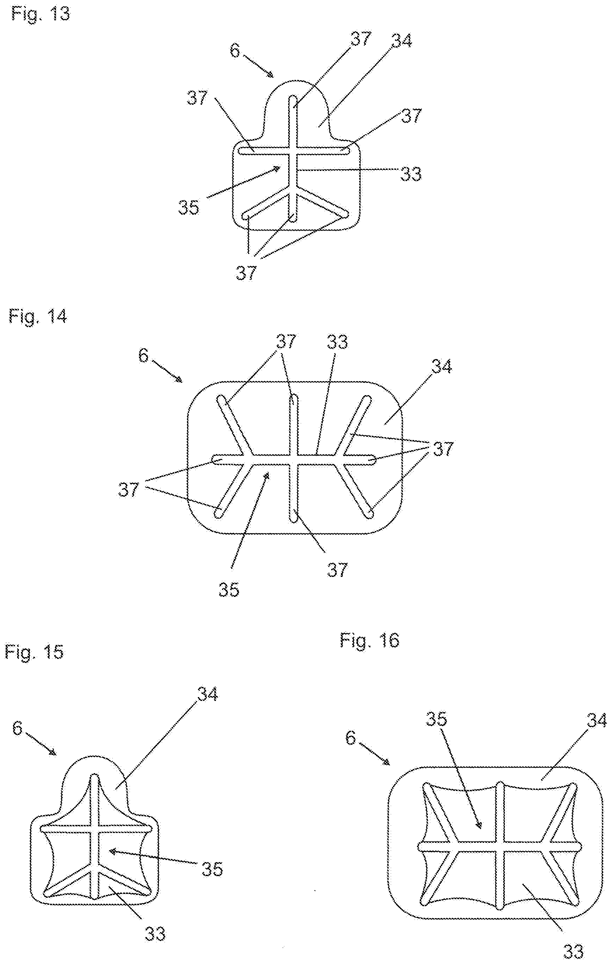

Figur 13 und Figur 14 zeigen zwei weitere Ausführungsformen einer vorgeschlagenen zweischichtigen Folie 6 mit unterschiedlichen äußeren Konturen der Deckschicht 34 und unterschiedlich ausgebildeten Formstrukturen 35 der Formschicht 33.

Figur 15 und Figur 16 zeigen weitere Beispiele von vorgeschlagenen Folien 6, wobei in den hier gezeigten Beispielen die Formschicht 33 jeweils als Gel auf die Deckschicht 34 aufgebracht wurde und in weiterer Folge aushärtete. Die hier gezeigten Formschichten 33 umfassen jeweils eine Formstruktur 35, die beispielsweise dadurch erzielt wurde, dass in den Bereichen der Formstruktur 35 mehr Gel aufgebracht wurde, sodass die Formschichten 33 unterschiedliche Schichtdicken aufweisen. Im Bereich einer Formstruktur 35 weist eine Formschicht 33 eine jeweils stärkere Schichtdicke auf als in den übrigen Bereichen der Formschicht 33.

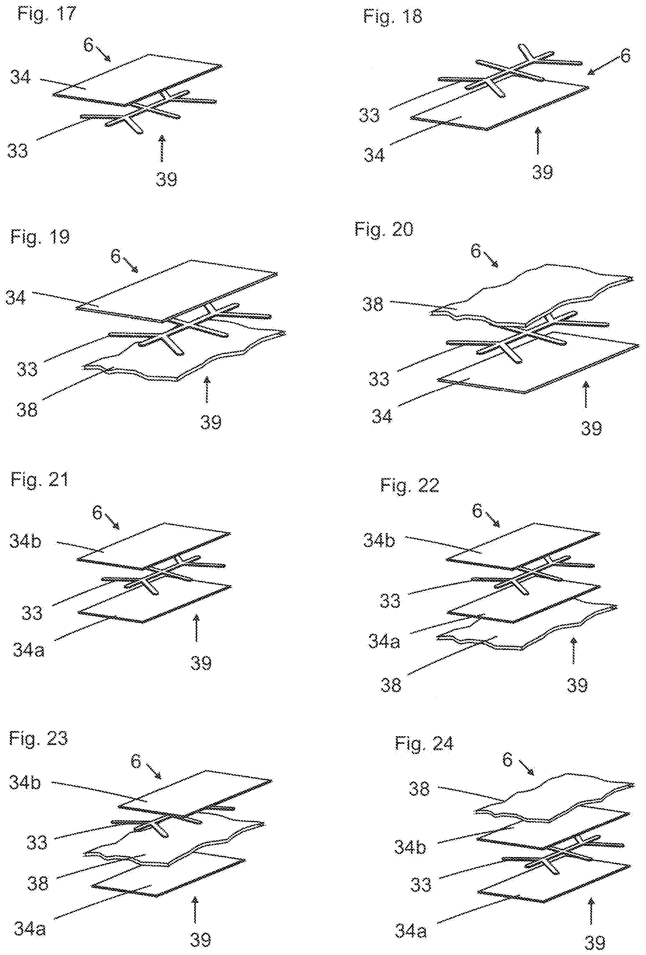

Figur 17 bis Figur 24 zeigen weitere Ausführungsbeispiele einer vorgeschlagenen Folie 6 in jeweils perspektivischer Explosionsdarstellung. Die in den Figuren jeweils nach unten weisende Seite 39 einer Folie 6 ist dabei die einer Knochendefektstelle 2 zuzuwendende Seite 39 der Folie 6.

Die Beispiele der Figur 17 und Figur 18 sind zweischichtig aufgebaut und umfassen jeweils eine Formschicht 33 und eine Deckschicht 34, wobei die Formschicht 33 eine geringere Flächenausdehnung als die Deckschicht 34 einnimmt. Die Beispiele der Figur 19 und Figur 20 sind dreischichtig aufgebaut und umfassen jeweils neben einer Formschicht 33 und einer Deckschicht 34 eine Trägerschicht 38, auf der Substanzen wie beispielsweise Medikamente, Wachstumsfaktoren und/oder sonstige die Heilung und Knochenbildung fördernde und schützende Substanzen aufgebracht sein können.

Die Beispiele der Figur 21 bis Figur 24 weisen jeweils eine Formschicht 33 und jeweils zwei Deckschichten 34a und 34b auf, wobei die Formschicht 33 eine geringere Flächenausdehnung als die Deckschichten 34a und 34b einnimmt. Die Beispiele der Figur 22 bis Figur 24 weisen jeweils zusätzlich eine Trägerschicht 38 auf, die mit entsprechenden Substanzen (wie oben zu Figur 19 und Figur 20 beschrieben) bestückt sein kann.

## Patentansprüche

1. Zahnimplantatsystem (1) für die Knochenregeneration einer Knochendefektstelle (2) eines Kieferknochens (3) umfassend
- ein im Kieferknochen (3) zu verankerndes Implantat (4) mit einer Gewindebohrung (5),
- eine Folie (6) zur Überdeckung der Knochendefektstelle (2) und des Implantats (4),
- eine Befestigungsschraube (7) zur lagestabilen Befestigung der Folie (6) relativ zum Implantat (4), wobei in Montageposition ein Schraubenbolzen (8) der Befestigungsschraube (7) durch die Folie (6) hindurch ragt und mit der Gewindebohrung (5) des Implantats (4) verschraubbar ist,
wobei ein Distanzelement (9) zwischen dem Implantat (4) und der Folie (6) anordenbar ist, wobei in Montageposition der Schraubenbolzen (8) durch das Distanzelement (9) hindurch ragt, **dadurch gekennzeichnet, dass** das Zahnimplantatsystem (1) eine im Wesentlichen rohrförmige Befestigungshülse (18) umfasst, durch die in Montageposition der Schraubenbolzen (8) hindurch ragt, wobei die Folie (6) zwischen Distanzelement (9) und Befestigungshülse (18) einklemmbar ist.

2. Zahnimplantatsystem nach Anspruch 1, **dadurch gekennzeichnet, dass** das Distanzelement (9) zum Hindurchführen des Schraubenbolzens (8) eine Durchgangsbohrung (10) mit einem Bohrungsdurchmesser (11) aufweist, der größer ist als ein Außendurchmesser (12) des Schraubenbolzens (8).

3. Zahnimplantatsystem nach Anspruch 1 oder 2, **dadurch gekennzeichnet, dass** das Distanzelement (9) am Implantat (4) rotationsgesperrt anordenbar ist, wobei vorzugsweise als Rotationssperre am Distanzelement (9) Vorsprünge (13) angeordnet sind, die in korrespondierende Ausnehmungen (14) des Implantats (4) einführbar sind.

4. Zahnimplantatsystem nach einem der Ansprüche 1 bis 3, **dadurch gekennzeichnet, dass** das Distanzelement (9) im Wesentlichen hohlzylinderförmig, vorzugsweise als im Wesentlichen rohrförmige Hülse, ausgebildet ist.

5. Zahnimplantatsystem nach einem der Ansprüche 1 bis 3, **dadurch gekennzeichnet, dass** das Distanzelement (9) im Wesentlichen konisch oder kegelstumpfförmig ausgebildet ist.

6. Zahnimplantatsystem nach einem der Ansprüche 1 bis 5, **dadurch gekennzeichnet, dass** die Befestigungsschraube (7) einen Schraubenkopf (15) aufweist, wobei die Folie (6) zwischen Distanzelement (9) und Schraubenkopf (15) einklemmbar ist.

7. Zahnimplantatsystem nach Anspruch 6, **dadurch gekennzeichnet, dass** der Schraubenkopf (15) an einer in Montageposition der Folie (6) zugewandten Schraubenkopfoberfläche (16) mit einer reibungsverringernden Beschichtung, vorzugsweise mit einer Teflonbeschichtung, versehen ist.

8. Zahnimplantatsystem nach Anspruch 6 oder 7, **dadurch gekennzeichnet, dass** die Folie (6) an einer in Montageposition dem Schraubenkopf (15) zugewandten Folienoberfläche (17) wenigstens in einem Kontaktbereich mit dem Schraubenkopf (15) mit einer reibungsverringernden Beschichtung, vorzugsweise mit einer Teflonbeschichtung, versehen ist.

9. Zahnimplantatsystem nach einem der Ansprüche 1 bis 8, **dadurch gekennzeichnet, dass** die Befestigungshülse (18) zum Hindurchführen des Schraubenbolzens (8) ein Durchgangsloch (19) mit einem Lochdurchmesser (20) aufweist, der größer ist als ein Außendurchmesser (12) des Schraubenbolzens (8).

10. Zahnimplantatsystem nach einem der Ansprüche 1 bis 9, **dadurch gekennzeichnet, dass** an der Befestigungshülse (18) an einer in Montageposition der Folie (6) zugewandten Hülsenoberfläche (21) wenigstens ein - vorzugsweise im Wesentlichen dornartig ausgebildeter - Fortsatz (22) zur Lagefixierung der Folie (6) relativ zur Befestigungshülse (18) angeordnet ist.

11. Zahnimplantatsystem nach einem der Ansprüche 1 bis 10, **dadurch gekennzeichnet, dass** die Befestigungshülse (18) als gesonderter Teil ausgebildet ist.

12. Zahnimplantatsystem nach einem der Ansprüche 1 bis 11, **dadurch gekennzeichnet, dass** die Folie (6) im Wesentlichen vollständig resorbierbar ist.

13. Zahnimplantatsystem nach einem der Ansprüche 1 bis 12, **dadurch gekennzeichnet, dass** die Folie (6) eine, vorzugsweise vorverbundene, mehrschichtige Folie ist, die eine formgebende Formschicht zur Anformung der Folie an die Knochendefektstelle (2) und wenigstens eine Deckschicht zur Abdeckung der Knochendefektstelle (2) umfasst, wobei die Formschicht und die wenigstens eine Deckschicht im Wesentlichen vollständig resorbierbar sind.

14. Zahnimplantatsystem nach Anspruch 13, **dadurch gekennzeichnet, dass** die Formschicht steifer als die wenigstens eine Deckschicht ausgebildet ist.

15. Zahnimplantatsystem nach Anspruch 13 oder 14, **dadurch gekennzeichnet, dass** die wenigstens eine Deckschicht zumindest teilweise, vorzugsweise im Wesentlichen vollständig, aus einem bioresorbierbaren Collagenmaterial besteht, wobei vorzugsweise das bioresorbierbare Collagenmaterial Typ-I-Collagen und/oder Typ-III-Collagen umfasst.

16. Zahnimplantatsystem nach einem der Ansprüche 13 bis 15, **dadurch gekennzeichnet, dass** die Formschicht zumindest teilweise, vorzugsweise im Wesentlichen vollständig, aus einem bioresorbierbaren Polymermaterial besteht, wobei vorzugsweise das bioresorbierbare Polymermaterial Milchsäure, vorzugsweise L-Milchsäure, und/oder deren Derivate umfasst.

## Claims

1. A dental implant system (1) for bone regeneration of a bone defect site (2) of a jawbone (3) including
- an implant (4) which is to be anchored in the jawbone (3) and has a threaded bore (5),
- a film (6) for covering over the bone defect site (2) and the implant (4), and
- a fixing screw (7) for positionally stable fixing of the film (6) relative to the implant (4), wherein in the fitted position a screw bolt (8) of the fixing screw (7) projects through the film (6) and can be screwed to the threaded bore (5) in the implant (4),
wherein a spacer element (9) can be arranged between the implant (4) and the film (6), wherein in the fitted position the screw bolt (8) projects through the spacer element (9), **characterised in that** the dental implant system (1) includes a substantially tubular fixing sleeve (18) through which the screw bolt (8) projects in the fitted position, wherein the film (6) can be clamped between the spacer element (9) and the fixing sleeve (18).

2. A dental implant system as set forth in claim 1 **characterised in that** the spacer element (9) for passing the screw bolt (8) therethrough has a through bore (10) of a bore diameter (11) larger than an outside diameter (12) of the screw bolt (8).

3. A dental implant system as set forth in claim 1 or claim 2 **characterised in that** the spacer element (9) can be arranged in rotationally locked relationship on the implant (4), wherein preferably provided as rotational locking means on the spacer element (9) are projections (13) which can be inserted into corresponding recesses (14) of the implant (4).

4. A dental implant system as set forth in one of claims 1 through 3 **characterised in that** the spacer element (9) is of a substantially hollow cylindrical shape, preferably in the form of a substantially tubular sleeve.

5. A dental implant system as set forth in one of claims 1 through 3 **characterised in that** the spacer element (9) is of a substantially conical or frustoconical configuration.

6. A dental implant system as set forth in one of claims 1 through 5 **characterised in that** the fixing screw (7) has a screw head (15), wherein the film (6) can be clamped between the spacer element (9) and the screw head (15).

7. A dental implant system as set forth in claim 6 **characterised in that** at a screw head surface (16) which in the fitted position is towards the film (6) the screw head (15) is provided with a friction-reducing coating, preferably with a Teflon coating.

8. A dental implant system as set forth in claim 6 or claim 7 **characterised in that** the film (6) is provided with a friction-reducing coating, preferably with a Teflon coating, at a film surface (17) that is towards the screw head (15) in the fitted position, at least in a contact region with the screw head (15).

9. A dental implant system as set forth in one of claims 1 through 8 **characterised in that** the fixing sleeve (18) for passing the screw bolt (8) therethrough has a through hole (19) of a hole diameter (20) which is larger than an outside diameter (12) of the screw bolt (8).

10. A dental implant system as set forth in one of claims 1 through 9 **characterised in that** provided on the fixing sleeve (18) at a sleeve surface (21) which in the fitted position is towards the film (6) is at least one extension (22) - preferably of a substantially thorn-like configuration - for positional fixing of the film (6) relative to the fixing sleeve (18).

11. A dental implant system as set forth in one of claims 1 through 10 **characterised in that** the fixing sleeve (18) is in the form of a separate part.

12. A dental implant system as set forth in one of claims 1 through 11 **characterised in that** the film (6) is substantially completely resorbable.

13. A dental implant system as set forth in one of claims 1 through 12 **characterised in that** the film (6) is a, preferably pre-bonded, multi-layer film which includes a shaping forming layer for shaping the film to the bone defect site (2) and at least one cover layer for covering the bone defect site (2), wherein the forming layer and the at least one cover layer are substantially completely resorbable.

14. A dental implant system as set forth in claim 13 **characterised in that** the forming layer is stiffer than the at least one cover layer.

15. A dental implant system as set forth in claim 13 or claim 14 **characterised in that** the at least one cover layer at least partially and preferably substantially completely comprises a bioresorbable collagen material, wherein preferably the bioresorbable collagen material includes type-I-collagen and/or type-III-collagen.

16. A dental implant system as set forth in one of claims 13 through 15 **characterised in that** the forming layer at least partially and preferably substantially completely comprises a bioresorbable polymer material, wherein preferably the bioresorbable polymer material includes lactic acid, preferably L-lactic acid, and/or derivatives thereof.

## Revendications

1. Système d'implant dentaire (1) pour la régénération osseuse d'un défaut osseux (2) d'un os maxillaire (3) comprenant
- un implant (4) à ancrer dans l'os maxillaire (3) avec un trou taraudé (5),
- une feuille (6) pour recouvrir le défaut osseux (2) et l'implant (4),
- une vis de fixation (7) pour la fixation en position stable de la feuille (6) par rapport à l'implant (4), dans lequel en position de montage, un boulon fileté (8) de la vis de fixation (7) pénètre à travers la feuille (6) et peut être vissé avec le trou taraudé (5) de l'implant (4),
dans lequel un élément d'écartement (9) peut être disposé entre l'implant (4) et la feuille (6), dans lequel en position de montage, le boulon fileté (8) pénètre à travers l'élément d'écartement (9), **caractérisé en ce que** le système d'implant dentaire (1) comprend une douille de fixation (18) essentiellement en forme de tube à travers laquelle pénètre le boulon fileté (8) en position de montage, dans lequel la feuille (6) peut être coincée entre l'élément d'écartement (9) et la douille de fixation (18).

2. Système d'implant dentaire selon la revendication 1, **caractérisé en ce que** l'élément d'écartement (9) pour le passage du boulon fileté (8) présente un trou de passage (10) avec un diamètre de perçage (11) qui est plus grand qu'un diamètre externe (12) du boulon fileté (8).

3. Système d'implant dentaire selon la revendication 1 ou 2, **caractérisé en ce que** l'élément d'écartement (9) peut être disposé en étant bloqué en rotation sur l'implant (4), dans lequel sont disposées sur l'élément d'écartement (9), de préférence comme blocage de rotation, des parties en saillie (13) qui peuvent être introduites dans des évidements correspondants (14) de l'implant (4).

4. Système d'implant dentaire selon l'une des revendications 1 à 3, **caractérisé en ce que** l'élément d'écartement (9) est conçu essentiellement en forme de cylindre creux, de préférence comme douille essentiellement en forme de tube.

5. Système d'implant dentaire selon l'une des revendications 1 à 3, **caractérisé en ce que** l'élément d'écartement (9) est conçu essentiellement de façon conique ou en forme de cône tronqué.

6. Système d'implant dentaire selon l'une des revendications 1 à 5, **caractérisé en ce que** la vis de fixation (7) présente une tête de vis (15), dans lequel la feuille (6) peut être coincée entre l'élément d'écartement (9) et la tête de vis (15).

7. Système d'implant dentaire selon la revendication 6, **caractérisé en ce que** la tête de vis (15) est prévue sur une surface de tête de vis (16) orientée vers la feuille (6) en position de montage, avec une enduction réduisant le frottement, de préférence avec une enduction en téflon.

8. Système d'implant dentaire selon la revendication 6 ou 7, **caractérisé en ce que** la feuille (6) est prévue sur une surface de feuille (17) orientée vers la tête de vis (15) en position de montage, au moins dans une zone de contact avec la tête de vis (15) avec une enduction réduisant le frottement, de préférence avec une enduction en téflon.

9. Système d'implant dentaire selon l'une des revendications 1 à 8, **caractérisé en ce que** la douille de fixation (18) pour le passage du boulon fileté (8) présente un trou de passage (19) avec un diamètre de trou (20) qui est plus qu'un diamètre externe (12) du boulon fileté (8).

10. Système d'implant dentaire selon l'une des revendications 1 à 9, **caractérisé en ce que** sur la douille de fixation (18), sur une surface de douille (21) orientée vers la feuille (6) en position de montage, est disposé au moins un prolongement (22) - conçu de préférence essentiellement en forme de pointe - destiné à la fixation en position de la feuille (6) par rapport à la douille de fixation (18).

11. Système d'implant dentaire selon l'une des revendications 1 à 10, **caractérisé en ce que** la douille de fixation (18) est conçue comme une partie séparée.

12. Système d'implant dentaire selon l'une des revendications 1 à 11, **caractérisé en ce que** la feuille (6) est résorbable essentiellement intégralement.

13. Système d'implant dentaire selon l'une des revendications 1 à 12, **caractérisé en ce que** la feuille (6) est une feuille à plusieurs couches, de préférence pré-reliée, qui comprend une couche moulée de modelage pour l'adaptation de la feuille au défaut osseux (2) et au moins une couche de recouvrement pour recouvrir le défaut osseux (2), dans lequel la couche moulée et la au moins une couche de recouvrement sont résorbables essentiellement intégralement.

14. Système d'implant dentaire selon la revendication 13, **caractérisé en ce que** la couche moulée est conçue plus rigide que la au moins une couche de recouvrement.

15. Système d'implant dentaire selon la revendication 13 ou 14, **caractérisé en ce que** la au moins une couche de recouvrement est composée au moins partiellement, de préférence essentiellement intégralement, d'un matériau collagénique bio-résorbable, dans lequel le matériau collagénique bio-résorbable comprend de préférence du collagène de type I et / ou du collagène de type III.

16. Système d'implant dentaire selon l'une des revendications 13 à 15, **caractérisé en ce que** la couche moulée est composée au moins partiellement, de préférence essentiellement intégralement, d'un matériau polymère bio-résorbable, dans lequel de préférence le matériau polymère bio-résorbable comprend un acide lactique, de préférence un acide lactique L, et / ou ses dérivés.
